# EUROPÄISCHE PATENTSCHRIFT

(11) **EP 0 776 194 B1**
(45) Veröffentlichungstag und Bekanntmachung des Hinweises auf die Patenterteilung: **02.05.2003**
(21) Anmeldenummer: 95944702.0
(22) Anmeldetag: 18.08.1995
(51) Int. Cl.: A61K 9/133, A61K 31/28

(54) **UNILAMELLARE LIPOSOMENZUBEREITUNGEN MIT HOHEM WIRKSTOFFANTEIL**
UNILAMELLAR LIPOSOMAL PREPARATIONS WITH HIGH ACTIVE SUBSTANCE CONTENT
PREPARATIONS LIPOSOMALES UNILAMELLAIRES, AVEC UNE TENEUR ELEVEE EN PRINCIPE ACTIF

(30) Priorität: 20.08.1994 DE 4430592
(43) Veröffentlichungstag der Anmeldung: 04.06.1997
(73) Patentinhaber: MAX-DELBRÜCK-CENTRUM FÜR MOLEKULARE MEDIZIN, 13125 Berlin (DE); Brandl, Martin, Prof. Dr., 9014 Tromso (NO)
(72) Erfinder: BRANDL, Martin, D-79102 Freiburg (DE); BACHMANN, Dieter, D-79110 Freiburg (DE); RESZKA, Regine, D-16341 Schwanebeck (DE); DRECHSLER, Markus, D-79110 Freiburg (DE)
(74) Vertreter: Baumbach, Friedrich, Dr.
(86) Internationale Anmeldenummer: DE9501162
(87) Internationale Veröffentlichungsnummer: WO96005808

(56) Entgegenhaltungen:
- EP-A- 0 509 338
- EP-A- 0 565 361
- J. LIPID RESEARCH, Bd. 21, 1980 Seiten 981-992, HAMILTON ET AL. 'Unilamellar liposomes made with the French pressure cell: a simple preparative and semiquantitative technique'
- LIPOSOME TECHNOLOGY, Bd. I, 1993 Seiten 37-48, G. GREGORIADIS ET AL. 'Efficient entrapment of solutes in microfluidized small dehydration-rehydration liposomes' in der Anmeldung erwähnt & GREGORIADIS (ED.) 'Liposome Technology' 1993 , CRC PRESS INC. , BOTA RATON, FLORIDA

## Beschreibung

Die Erfindung betrifft eine liposomale Zubereitung, ihre Herstellung und ihre Verwendung. Anwendungsgebiete sind die Pharmazie und die Medizin, insbesondere die Verwendung als Trägersysteme fiir Arzneistoffe, Impfstoffe, Diagnostika und Vektoren.

Liposomen haben in den letzten Jahren eine ständig steigende Bedeutung als Trägersysteme bzw. Verkapselungsmittel für verschiedene Stoffe, insbesondere für medizinische Anwendungen erlangt (vgl. Liposomes from Physics to Applications, D. D. Lasic, Eisevier Amsterdam 1993). Dabei werden vor allem solche Liposomen als vorteilhaft angesehen, die eine einheitliche kleine bis mittlere Vesikelgröße (50 bis 300 nm) sowie bevorzugt eine einzige Membranschicht (unilamellare Vesikel) aufweisen. Darüber hinaus sollen die Wirkstoffe effizient in die Liposomen eingeschlossen oder inkorporiert werden.
Für die Herstellung von Liposomen sind zahlreiche Verfahren beschrieben. Diese Herstellungsverfahren erfüllen jedoch jeweils eine der beiden o. g. zentralen Forderungen, kleine bis mittlere Größe bzw. hohe Einschlußeffizienz, nur unzureichend. Dies gilt besonders dann, wenn es sich um wasserlösliche Wirkstoffe handelt, die im wäßrigen Innenraum der Liposomen verkapselt vorliegen sollen. So führen z. B. die DRV-Methode und die REV-Methode bei guter Einschlußeffizienz zu Populationen uneinheitlicher, überwiegend sehr großer Liposomen. Dagegen erreichen bekannte Präparatiosmethoiden auf der Basis von Ultraschallbehandlung, Hochdruckhomogenisation oder Detergent-dialyse bei einheitlich kleinen bis mittleren Liposomengrößen nur mäßige Einschlußeffizienz vor allem bei wasserlöslichen Substanzen (Liposome Technology 2nd edition, volume I-III, G. Gregoriadis (Ed.), CRC Press Inc., Boca Raton, Florida, 1993, 37-48; D. Bachmann et al, Preparation of Liposomes using a Mini-Lab 8.30 H high-pressure homogenizer, Int. J. Pharm.91, 1/93, 69-74).

So werden beispielsweise im Dokument EP-A-509338 unilamellare Liposomen beschrieben, die eine Vesikelgröße von 20-50 nm mit einem Wirkstoffanteil bis 33% haben. Sie werden mittels Hochdruckhomogenisation produziert. Dabei handelt es sich ausschließlich um Liposomenzubereitungen aus Rohstoffgemischen, die fettsäreverestertes Collagenhydrolysat enthalten, damit kleine Liposomendurchmesser entstehen. Da das Liposomengel erst nach dem Zusatz von Gelbildnern erhalten wird, liegen sie nicht in dichter Packung vor. Eine Verkapselung von Wirkstoffen wird nicht erwähnt. Die genannten Wirkstoffe sind außerdem lipophil, so daß hier keine Verkapselung vorliegt.

Die im Dokument EP-A-565361 beschriebenen Liposomen haben eine Vesikelgröße von etwa 100 nm. Der Wirkstoffgehalt beträgt schätzungsweise 20 % bei einer 5 fachen Osmolarität. Es handelt sich dabei jedoch nicht um kleine einheitliche Liposomen mit einer gleichzeitigen hohen Verkapselung für Wirkstoffe und dichter Packung. Sie werden aus Lezithin mit 1-90% fettsäreverestertem Collagenhydrolysat zubereitet. In dem zitierten Dokument erfolgt die Stabilisierung der Liposomen durch hohen osmotischen Druck, so daß ein Unterschied zwischen dem wässrigen Innenraum der Liposomen und dem bestehenden Medium besteht, wodurch die Höhe der Wirkstoffkonzentration begrenzt ist.

Die von HAMILTON u.a. im J. Lipid Research (1980, BD. 21, S. 981-992) beschriebenen unilamellaren Liposomen sind im Mittel 20 nm groß und bestehen aus gelöstem oder in wässriger Lösung konzentriertem Ei-phosphatidylcholin. Sie werden mittels Hochdruckhomogenisation in einer French Pressure Kammer hergestellt. Dabei ist die Lipidkonzentration niedrig und beträgt nur 25 mg/ml. Dadurch können keine dichtgepackten Liposomenzubereitungen erreicht werden. Die Homogenität der Liposomengrößen ist nicht so optimal, eine Verkapselung von Wirkstoffen wird nicht erwähnt.

Die Erfindung hat das Ziel, eine liposomale Zubereitung auf der Basis einheitlich kleiner bis mittlerer Liposomen mit hoher Einschlußeffizienz für Wirkstoffe herzustellen.

Die Erfindung wird gemäß den Patentausprüchen 1 und 2 realisiert. Die Unteransprüche sind Vorzugsvarianten.
Die erfindungsgemäße liposomale Zubereitung besteht aus einheitlichen kleinen bis mittelgroßen, meist unilamellaren Liposomen mit einer Größe von 10 bis 300 um Durchmesser. Sie hat eine vesikuläre Struktur in dichter Packung und enthält einen hohen Anteil Wirkstoff, mindestens 20% und in einigen Fällen bis zu 90%, in liposomal verkapselter Form. Unter Wirkstoff versteht man arzneilich wirksame Stoffe (Arzneistoffe), insbesondere Cytostatika, Antibiotika, Peptide und Proteinen; sowie Stoffe, die ggf. am oder im Körper angewandt werden zur Erkennung seiner Funktion oder Beschaffenheit oder der seiner Organe oder Gewebe (Diagnostika); Stoffe, die zu einer Immunisierung gegen Krankheiten führen (Impfstoffe); Stoffe, die im Rahmen einer Gentherapie zur Übertragung von genetischen Material führen (Vektoren). Nur geringe Anteile des Wirkstoffs sind in freier nichtliposomaler Form vorhanden.
Die Zubereitung ist ein hochviskoses, fast klares bis opaleszierendes oder leicht trübes Gel, das neben (Phospho)lipiden oder anderen geeigneten amphiphilen membranbildenden Substanzen auch Arzneistoffe, Diagnostika, Vakzine oder auch Elektrolyte wie z. B. solche zur Einstellung eines bestimmten osmotischen Drucks oder eines bestimmten pH-Werts. Als Lipide werden z.B. Cholesterol und dessen Analoga, Phospholipide natürlichen wie synthetischen Ursprungs, wie z.B. Phosphatidylcholin, Phosphatidylglycerol und deren Analoga eingesetzt. Andere membranbildende Amphiphile sind synthetische Amphiphile wie z.B. Blockpolymere, Alkylester, -ether, -amide von Alkoholen, Diolen, Triolen und Polyolen, Aminen, Aminosäuren, Peptiden und Zuckern. Die Liposomen sind so dicht gepackt, daß sie nur einen geringen Zwischenraum zwischen den Vesikeln ausbilden, ohne daß jedoch ihre vesikuläre Struktur gestört wäre. Man bezeichnet diese Struktur auch als Liposomengel.

Dieses Liposomengel kann nach der Herstellung und vor der schrittweisen Verdünnung bei Raumtemperatur gelagert werden. Da die erzielten relativen Einschlüsse in die vorgebildeten Liposomen vergleichsweise hoch sind, kann zumindest für bestimmte Anwendungen auf eine Abtrennung des nichteingeschlossenen Wirkstoffs verzichtet werden. In der Folge herrscht somit in dem erhaltenen Liposomengel innerhalb wie außerhalb der Liposomen eine gleich hohe konzenztration an gelöstem Wirkstoff, so daß ein Austreten des verkapselten Wirkstoffs aus den Liposomen nicht auftritt. Durch Einbau in einen dreidimensionalen Gelverband weisen die Liposomen außerdem hinsichtlich ihrer Größe und Lamellarität eine gute Lagerstabilität auf. Dies ist darauf zurückzuführen, daß die gebundenen Liposomen ein geringere Beweglichkeit, d.h. geringere kinetische Energie aufweisen und somit die erforderliche kinetische Stoßenrgie zur Aggregation und Fusion der Liposomen weniger leicht erreicht wird. In den Fällen, in denen die Liposomenzubereitung Gefrier-Tau-Zyklen unterworfen wird, ist außerdem eine Lagerung nach dem letzten Einfriervorgang bei Tiefkühltemperaturen und Auftauen kurz vor Gebrauch vorteilhaft.

Die beanspruchte liposomale Zubereitung hat drei wesentliche Vorteile gegenüber den bisher bekannten Zubereitungen:
- Aufnahme bis zur 20fachen Wirkstoffmenge (sonst werden nur 1 bis 5 % Verkapselungseffizienz erreicht) in kleine bis mittelgroße unilamellare Liposomen.
- Hohe Stabilität, die Lagerfähigkeit beträgt mindestens 3 Jahre.
- Einheitliche Größe der Liposomen, Abweichung mehr als 95% der Liposomen haben typischerweise Durchmesser innerhalb einer Spannweite von 100 nm.

Die Zubereitungen können direkt als Depot des verkapselten Stoffes angewendet, z. B. injiziert bzw. implantiert werden.

Alternativ können aus diesen Zubereitungen mittels schrittweiser Zugabe von wäßrigem Medium ggf. unter Mischung mit geringer mechanischer Belastung, z. B. durch Schütteln oder Rühren, Liposomen freigesetzt werden, die kleine bis mittlere Größen sowie nur wenige (bevorzugt eine) konzentrische Lamellen aufweisen und die unverändert einen hohen Prozentsatz des zugegebenen Wirkstoffs in verkapselter Form enthalten.

Wäßriges Medium ist hauptsächlich "gereinigtes Wasser" nach Deutschem Arzneibuch (im Falle von Zubereitungen zur parenteralen Anwendung " Wasser für Injektionszwecke" nach Deutschem Arzneibuch), in dem anorganische und organische Salze, Säuren oder Basen gelöst sein können, z.B. zur Einstellung eines bestimmten pH-Werts oder eines bestimmten osmotischen Drucks, aber auch wäßrige Lösungen von Zuckern, Aminosäuren und Gemische von Wasser mit anderen Lösungsmitteln wie Alkoholen, Diolen oder Triolen kommen in Betracht. Geeignet ist zum Beispiel 0,9%ige Natriumchloridlösung oder 40 mmol Phophatpuffer pH 7,4. Auch diese Zubereitungen verschiedensten Verdünnungsgrades, vom Liposomengel bis zur freifließenden Dispersion, sind Gegenstand dieser Erfindung.

Das erfindungsgemäße Herstellungsverfahren für die Zubereitungen nach den Ansprüchen 1 und 2 umfaßt folgende Schritte:
1. Vormischung
   Mischung von Lipid(en), Wasser und einzuschließenden Wirkstoffen (erforderlichenfalls grob dispergiert durch Rühren mittels eines Magnetrührers o. ä.)
2. Lipidfilm
   Alternativ Herstellung eines Lipidfilms und Überführung desselben in eine Vormischung bestehend aus (Phospho)lipid(en) und wäßrigem Medium. Der dünne, trockene Lipidfilm wird durch Auflösen des Lipids oder der Lipide in organischen Lösungsmitteln, die ggf. zu inkorporiende Sustanzen enthalten, und nachfolgendes Entfernen des Lösungsmittels durch Evaporation oder Sprühtrocknung erhalten. Er wird dann nach Zugabe von wäßrigem Medium, das ggf. einzuschließende Substanzen enthält, durch Schütteln, Rühren, Kneten o. ä. dispergiert. Dabei wird die Temperatur erforderlichenfalls auf ca. 10° C oberhalb der Phasenübergangstemperatur der Phospholipide erhöht.
3. Hochdruckhomogenisation
   Die Vormischung aus 1 oder 2 wird ein- oder mehrmals (jedoch nicht mehr als fünfzigmal) der Hochdruckhomogenisation unter Drucken zwischen 50 und 1600 bar (5 bis 160 MPa) unterzogen. Edukt, Homogenisator und Produkt werden ggf. temperiert.
4. Fakultativ Gefrier/Tau-Behandlung
   Durch ein- bis mehrmaliges Einfrieren und nachfolgendes Wiederauftauen der Zubereitung während, d. h. zwischen den einzelnen Zyklen der Hochdruckhomogenisation oder an deren Ende wird eine homogenere Durchmischung des Ansatzes erreicht und evtl. die vorgebildeten Liposomen vorübergehend destabilisiert, so daß Liposomen mit noch mehr eingeschlossener Substanz gebildet werden können.
5. Fakultativ Überführung in freifließende Liposomendispersion
   Durch schrittweise Zugabe von wäßrigem Medium in Volunmenanteilen von 1 bis 30% des Volumens der Zubereitung und mechanisches Mischen durch Schütteln oder Rühren wird das dreidimensionale Gelgerüst zerlegt und werden die vorgebildeten Liposomen freigesetzt.
6. Fakultativ Begrenzung der Liposomengröße nach oben und Abtrennung des unverkapselten Wirkstoffs
   Für bestimmte Anwendungsfälle ist es zweckmäßig, eventuell vereinzelt vorkommende größere Liposomen oder Lipidaggregate durch Filtration der Zubereitung durch Membranfilter mit einer Porenweite von 0,1 bis 1 µm abzutrennen bzw. zu zerkleinern. Die Membranfiltration trennt zudem etwa vorhandene Bakterien ab und dient so zur Herstellung einer sterilen Zubereitung. Überschüssiger Wirkstoff, der im Verlauf der Herstellung nicht in Liposomen eingeschlossen wurde, kann von diesen mittels Gelpermeationschromatographie, Dialyse, Ultrafiltration oder Ultrazentrifugation abgetrennt werden.

Die erfindungsgemäße Zubereitung, die Liposomen in dichter Packung enthält (Liposomengel), eignet sich als Wirkstoffdepot, das den Wirkstoff langsam freisetzt und zwar entweder in gelöster Form oder in Form einzelner Wirkstoff-enthaltender Liposomen. Diese Zubereitung kann durch Injektion (z.B. i.m., i.p.) oder Implantation angewendet werden. Aber auch ein Einbringen in Körperhöhlen oder ein Aufbringen auf Schleimhäute, auf die Hornhaut des Auges (Kornea) oder Hautpartien ist möglich. Somit dient die Zubereitung als Träger des Wirkstoffs sowie ggf. zu dessen modifizierter Freigabe.
Die erfindungsgemäße Zubereitung, die Liposomen in freifließender Form enthält, eignet sich als Träger des Wirkstoffs. Diese Zubereitung kann durch Injektion (z.B. i.v., i.m) oder ebenfalls durch Einbringen in Körperhöhlen oder Aufbringen auf Schleimhäute, auf die Hornhaut des Auges (Kornea) oder Hautpartien angewendet werden. Die erfindungsgemäß verkapselten Liposomen führen zu einer Verteilung des liposomalen Wirkstoffs im Körper, die selektiv eine hohe und lange andauernde Wirkstoffkonzentration am Wirkort bewirkt und damit zu einer Verbesserung der Wirkung oder zu einer Verbesserung des Verhältnisses von Wirkung zu Nebenwirkung

Die Erfindung soll nachfolgend durch Ausführungsbeispiele näher erläutert werden.

### Beispiel 1)

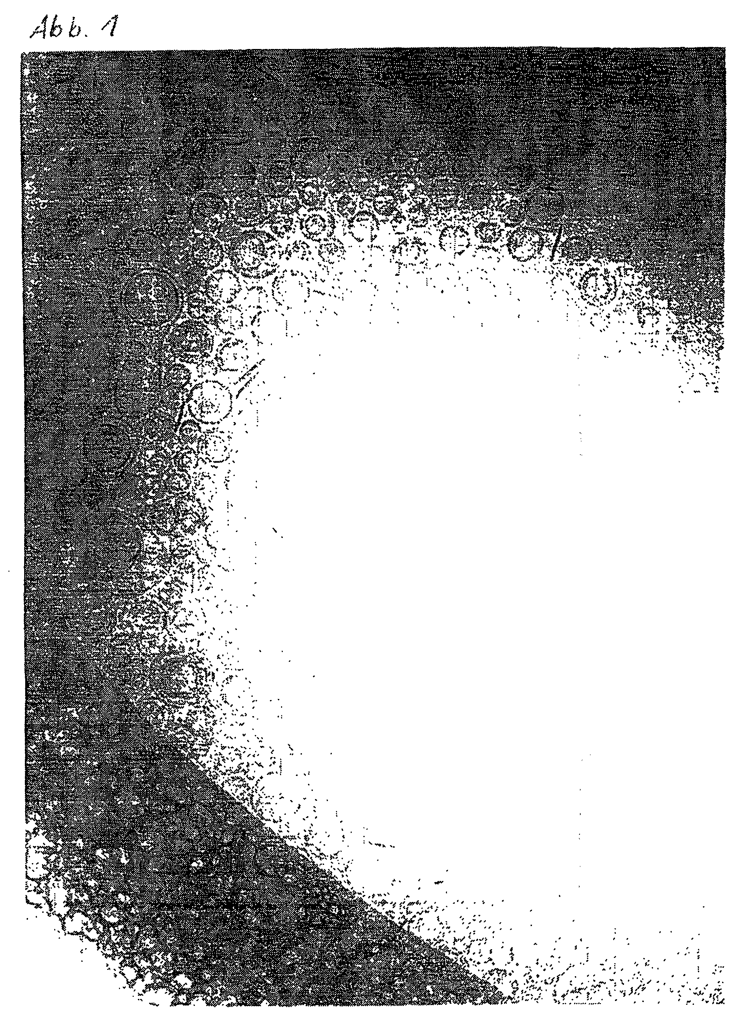

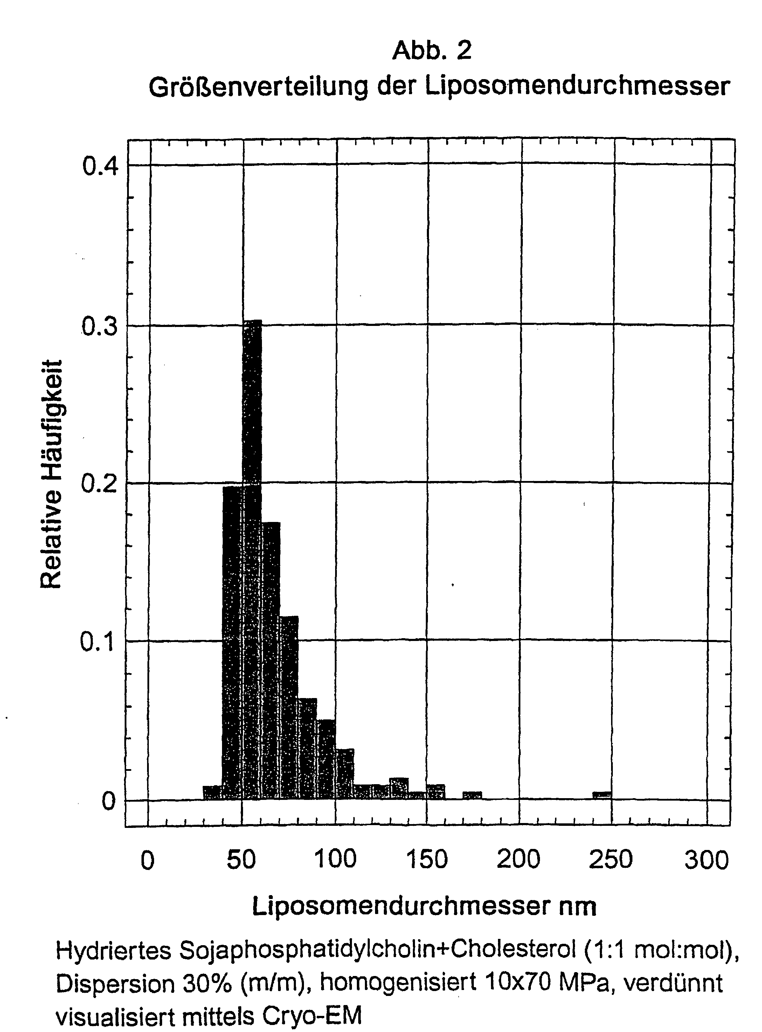

a) Herstellung eines Lipidfilms und Überführung desselben in eine Vormischung bestehend aus (Phospho)lipid(en) und wässrigem Medium:
   Herstellung einer Lipidlösung durch Auflösen von 5,96 Gramm hydrierten Sojaphosphatidylcholins zusammen mit 3,04 Gramm Cholesterol (entsprechend einem molaren Verhältnis der beiden Membranbildner von 1:1) in Chloroform. Nachfolgend vollständige Entfernung des Chloroforms durch Evaporation am Rotavapor bei ca. 40° bis 60° C und vermindertem Druck zwischen 400 und 20 hPa. Dispergieren des Lipidfilms nach Zugabe von 21 Millilitern einer ca. 0,1 millimolaren wäßriger Lösung des niedermolekularen hydrophilen Markers 5,6 Carboxyfluorescein in 40 millimolarem Phosphatpuffer (pH 7,4) durch manuelles Schütteln, unter erhöhter Temperatur (ca. 60 bis 70 ° C).
b) Hochdruckhomogenisation.
   Die aus a) erhaltene Vormischung wird zehnmal der Hochdruckhomogenisation (mittels APV Gaulin Micron Lab 40) unter Drucken von 70 MPa unterzogen. Edukt, Homogenisator und Produkt werden temperiert auf oberhalb ca. 60° C.
c) Überführung der Zubereitung in eine freifließende Liposomendispersion.
   Durch schrittweise Zugabe von wässrigem Medium in Volumenanteilen von 10% des Volumens der unter b) erhaltenen Zubereitung und mechanisches Mischen z.B. durch manuelles Schütteln sowie mittels eines Vortex Vibrationsmischers wird das dreidimensionale Gelgerüst zerlegt und die vorgebildeten Liposomen freigesetzt.
d) Abtrennung und Gehaltsbestimmung des liposomal verkapselten 5,6-Carboxyfluoresceins und des unverkapselten Markers zur Bestimmung der Verkapselungseffizienz
   Die Abtrennung der Liposomen erfolgt mittels Gelpermeationschromatographie auf einer Säule, die gefüllt ist mit vorgequollenem Sephadex G25 und Elution mit 40 millimolarem Phosphatpuffer (pH 7,4). Die 5,6-Carboxyfluoresceinmenge in der Liposomenfraktionen und der Fraktion, die das freie 5,6-Carboxyfluorescein enthält, wird quantitativ fluorimetrisch bestimmt bei einer Anregungswellenlänge von ca. 486 nm und einer Emissionswellenlänge von ca. 516 nm. Eine eventuelle Trübung hervorgerufen durch die Liposomen, die die Analyse behindert, wird beseitigt durch Zugabe einer 25%igen Lösung von Gallensalzen. Die Auswertung erfolgt gegen eine Kalibrierreihe verdünnter 5,6-Carboxyfluoreceinlösungen in 40 millimolarem Phosphatpuffer (pH 7,4) bekannten Gehalts. Die derart ermittelten relativen Anteile an liposomal verkapseltem im Verhältnis zum insgesamt in der Liposomendispersion enthaltenen Marker 5,6-Carboxyfluorecein (Verkapselungseffizienz) betragen ca. 40 %.
e) Elektronenmikroskopische Visualisierung und Größenanalyse der Liposomen
   Die nachfolgende Abbildung zeigt eine Aufnahme der unter c) erhaltenen Liposomen, die mittels Cryo-Elektronenmikroskopie angefertigt wurde. Aus der Abbildung 1 ist ersichtlich, daß die Liposomen als einzelne Partikel vorliegen und in ihrer weit überwiegenden Mehrzahl von kleiner und einheitlicher Größe und einschalig (unilamellar) sind. Das Ergebnis einer Größenanalyse von mehr als 400 der abgebildeten Liposomen ist im nachfolgenden Histogramm wiedergegeben (Abb 2).

### Beispiel 2)

a) Hochdruckhomogenisation.
   12 Gramm Sojaphosphatidylcholin und 18 Milliliter einer ca. 0,1 millimolaren wäßrigen Lösung des niedermolekularen hydrophilen Markers 5,6 Carboxyfluorescein in 40 millimolarem Phosphatpuffer (pH 7,4) werden durch manuelles Schütteln gemischt. Die erhaltene Mischung wird zehnmal der Hochdruckhomogenisation (mittels APV Gaulin Micron Lab 40) unter Drucken von 70 MPa unterzogen.
b) Gefrier-Tau-Vorgänge
   Die unter a) erhaltene gelartige Zubereitung wird bei Temperaturen von -10° C oder darunter eingefroren und danach bei Temperaturen von 60° C oder darüber aufgetaut. Dieser Gefrier-Tau-Vorgang wird nacheinander insgesamt dreimal durchgeführt.
c) Überführung der Zubereitung in eine freifließende Liposomendispersion.
   wie in Beispiel 1)
d) Abtrennung und Gehaltsbestimmung des liposomal verkapselten 5,6-Carboxyfluoresceins und des unverkapselten Markers zur Bestimmung der Verkapselungseffizienz
   wie in Beispiel 1)
   Die derart ermittelten relativen Anteile an liposomal verkapseltem im Verhältnis zum insgesamt in der Liposomendispersion enthaltenen Marker 5,6-Carboxyfluorecein (Verkapselungseffizienz) betragen ca. 80 %.

### Beispiel 3)

a) Herstellung eines Lipidfilms und Überführung desselben in eine Vormischung bestehend aus (Phospho)lipid(en) und wässrigem Medium:
   Herstellung einer Lipidlösung durch Auflösen von 4,42 Gramm Sojaphosphatidylcholin zusammen mit 0,535 Gramm Cholesterol (entsprechend einem molaren Verhältnis der beiden Membranbildner von 1:0,25) in Chloroform. Nachfolgend vollständige Entfernung des Chloroforms durch Evaporation am Rotavapor bei ca. 40° bis 60° C und vermindertem Druck zwischen 400 und 20 hPa. Dispergieren des Lipidfilms nach Zugabe von 25 Millilitern einer ca. 0,68 millimolaren wäßriger Lösung des niedermolekularen hydrophilen Zytostatikums Carboplatin.
b) Hochdruckhomogenisation.
   Die aus a) erhaltene Vormischung wird zehnmal der Hochdruckhomogenisation (mittels APV Gaulin Micron Lab 40) unter Drucken von 70 MPa unterzogen.
c) Gefrier-Tau-Vorgänge
   wie in Beispiel 2(b)
d) Überführung der Zubereitung in eine freifließende Liposomendispersion.
   wie in Beispiel 1(c)
e) Abtrennung und Gehaltsbestimmung des liposomal verkapselten Carboplatins
   Die Abtrennung der Liposomen, hergestellt nach Beispiel 3b, erfolgt mittels Gelpermeationschromatographie auf einer Säule, die gefüllt ist mit vorgequollenem Sephadex G25 und Elution mit isotonem Phosphatpuffer (pH 7,4).
   Liposomen, die nach Beispiel 3c präpariert wurden, werden dreimal mit je 100 ml isotonem Phosphatpuffer (pH 7,4) versetzt und dreißig Minuten bei 14 000 U/min zentrifugiert.
   Die Bestimmung des verkapselten Carboplatinanteils erfolgt mittels Hochdruckflüssigkeits-Chromatographie (HPLC) (Fichtner, I., Reszka, R., Schütt, M., Rudolph, M., Lemm, M., Richter, J., Berger, I., Carboplatin-Liposomes as Activators of Hematopoiesis. Oncology Research5: 65-74, 1993)
   Die derart ermittelte Verkapselungseffizienz beträgt ca. 40% (des eingesetzten Carboplatins)

### Beispiel 4)

4,4 Gramm Sojaphosphatidylcholin und 20 Milliliter einer ca. 0,94 millimolaren wäßrigen Lösung des niedermolekularen hydrophilen Zytostatikums werden durch manuelles Schütteln gemischt. Die erhaltene Mischung wird zehnmal der Hochdruckhomogenisation (mittels APV Gaulin Micron Lab 40) unter Drucken von 70 MPa unterzogen.
b) Gefrier-Tau-Vorgänge
   die unter a) erhaltene gelartige Zubereitung wird wie in Beispiel 2b) behandelt.
c) Überführung der Zubereitung in eine freifließende Liposomendispersion.
   wie in Beispiel 1).
d) Abtrennung und Gehaltsbestimmung des liposomal verkapselten Carboplatins
   wie in Beispiel 3e)
   Die derart ermittelte Verkapselungseffizienz beträgt ca. 44% (des eingesetzten Carboplatins)

## Patentansprüche

1. Liposomale Zubereitung, bestehend aus Liposomen als Träger von Wirkstoffen, wie Arzneistoffen, Impfstoffen, Diagnostika sowie Vektoren,
**dadurch gekennzeichnet,**
**dass** sie aus einheitlichen unilamellaren Liposomen kleiner bis mittlerer Größe mit 10 bis 300 nm Durchmesser, mit vesikulärer Struktur in dichter Packung besteht, mindestens 20% des Wirkstoffs in verkapselter Form enthält und ein hochviskoses Liposomengel darstellt.

2. Verfahren zur Herstellung der liposomalen Zubereitung nach Anspruch 1,
**dadurch gekennzeichnet,**
**dass** ein Gemisch von membranbildenden Amphiphilen, Wasser und einzuschließendem Wirkstoff, gegebenenfalls nach Herstellung eines dünnen, trockenen Lipidfilms, nachfolgender Entfernung der Lösungsmittel durch Evaporation oder Sprühtrocknung sowie nachfolgendes Dispergieren in Wasser, ein- oder mehrmals, höchstens fünfzigmal, einer Hochdruckhomogenisation mit Drucken von 50 bis 1600 bar (5 - 160 MPa) unterzogen wird und gegebenenfalls nachfolgend eine Gefrier/Tau-Behandlung, gegebenenfalls eine Überführung in eine freifließende Dispersion und anschließend gegebenenfalls eine Extrusion durch Filter mit einer Porenweite von 0,1 bis 1 µm erfolgt.

3. Verfahren nach Anspruch 2,
**dadurch gekennzeichnet,**
**dass** als membranbildende Amphiphile Lipide, Phospholipide natürlichen oder synthetischen Ursprungs oder synthetische Amphiphile eingesetzt werden.

4. Verfahren nach Anspruch 2 und 3,
**dadurch gekennzeichnet,**
**dass** als Lipid Cholesterol eingesetzt wird.

5. Verfahren nach Anspruch 2 und 3,
**dadurch gekennzeichnet,**
**dass** als Phospholipid Phosphatidylcholin oder Phosphatidylglycerol eingesetzt wird.

6. Verfahren nach Anspruch 2 und 3,
**dadurch gekennzeichnet,**
**dass** als synthetische Amphiphile Blockcopolymere, Alkylester, -ether und -amide von Alkoholen, Diolen, Triolen oder Polyolen, Aminen, Aminosäuren, Peptiden und Zuckern eingesetzt werden.

7. Verwendung einer liposomalen Zubereitung nach Anspruch 1 als Träger für Arzneistoffe, Impfstoffe, Diagnostika und Vektoren,
wobei die liposomale Zubereitung **dadurch gekennzeichnet ist,**
**dass** die Liposomen im wässrigen Medium freigesetzt werden unter Beibehaltung des hohen Anteils, vorzugsweise mindestens 20%, des Wirkstoffs in verkapselter Form.

## Claims

1. Liposomal preparation, comprising liposomes as the carriers of effective agents such as medications, vaccines, diagnostica and vectors,
wherein
it comprises standardised, unilamellar liposomes of a small to medium size with a diameter of 10 to 300 nm with a vesicular structure in a tight packing, contains at least 20% of the effective agent in an encapsulated form and portrays a highly viscous liposome gel.

2. Method for the production of the liposomal preparation according to Claim 1,
wherein
a mixture of membrane-forming amphiphiles, water and effective agent to be enclosed, if need be after production of a thin, dry lipid film, subsequent removal of the solvent by evaporation or spray drying and subsequent dispersion in water one or a number of times, fifty times at the most, is subjected to a high-pressure homogenisation with pressures between 50 and 1600 bar (5 - 160 MPa) and, if need be, a deep-freeze/thaw treatment, if need be conversion into a freely flowing dispersion and subsequently, if need be, an extrusion though filters with a pore width of 0.1 to 1 µm takes place.

3. Method according to Claim 2,
wherein
lipids, phospholipids of natural or synthetic origin or synthetic amphiphiles are used as the membrane-forming amphiphiles.

4. Method according to Claims 2 and 3,
wherein
cholesterol is used as a lipid.

5. Method according to Claims 2 and 3,
wherein
phosphatidylcholine or phosphatidylglycerol is used as a phospholipid.

6. Method according to Claims 2 and 3,
wherein
block copolymers, alkyl ester, ether and amides of alcohols, diols, triols or polyols, amines, amino acids, peptides and sugars are used as synthetic amphiphiles.

7. Use of a liposomal preparation according to Claim 1 as a carrier for medications, vaccines, diagnostica and vectors,
wherein
the liposomes are released in the watery medium, maintaining the high share, preferably at least 20%, of the effective agent in an encapsulated form.

## Revendications

1. Préparation liposomale, composée de liposomes en tant que supports de principes actifs, tels que des médicaments, des vaccins, des diagnostiques ainsi que des vecteurs.
se caractérisant par le fait
qu'elle se compose de liposomes uni-lamellaires homogènes de taille petite à moyenne avec 10 à 300 nm de diamètre, à structure vésiculaire en paquet compact, qu'elle contient au moins 20 % du principe actif sous forme capsulée et représente un gel liposomal à viscosité très élevée.

2. Procédé de fabrication de la préparation liposomale selon la revendication 1,
se caractérisant par le fait
qu'un mélange d'amphiphiles membranaires, d'eau et de principe actif à occlure, le cas échéant après la fabrication d'un film lipidique fin et sec, suivie de l'élimination du solvant par évaporation ou séchage par atomisation ainsi que suivie d'une hydrodispersion, sera soumis une ou plusieurs fois, au maximum cinquante fois, à une homogénéisation haute pression à des pressions de 50 à 1600 bar (5 - 160 MPa) et le cas échéant suivie d'un traitement gel/dégel, le cas échéant suivi d'un transfert dans une dispersion à écoulement libre et finalement suivi le cas échéant d'une extrusion au travers d'un filtre à pores de 0,1 à 1 µm.

3. Procédé selon la revendication 2,
se caractérisant par le fait
que des lipides, des phospholipides d'origine naturelle ou synthétique ou des amphiphiles synthétiques sont utilisés en tant qu'amphiphiles membranaires.

4. Procédé selon la revendication 2 et 3,
se caractérisant par le fait
que le cholestérol est utilisé en tant que lipide.

5. Procédé selon la revendication 2 et 3,
se caractérisant par le fait
que de la phosphatidylcholine ou du phosphatidylglycérol est utilisé en tant que phospholipide.

6. Procédé selon la revendication 2 et 3,
se caractérisant par le fait
que des copolymères en bloc, des alkylesters, alkyléthers et alkylamides d'alcools, de diols, de triols ou de polyols, des amines, des acides aminés, des peptides et des sucres sont utilisés en tant qu'amphiphiles synthétiques.

7. Emploi d'une préparation liposomale selon la revendication 1 en tant que support pour des médicaments, des vaccins, des diagnostiques et des vecteurs,
cette préparation liposomale se caractérisant par le fait
que les liposomes sont libérés dans un milieu aqueux tout en préservant une part élevée, de préférence au moins 20 % du principe actif sous forme capsulée.
